# EUROPEAN PATENT APPLICATION

(11) **EP 2 625 998 A1**
(43) Date of publication of application: **14.08.2013**
(21) Application number: 11830347.8
(22) Date of filing: 28.09.2011
(51) Int. Cl.: A61B 1/00, A61B 1/04

(54) **ENDOSCOPE**

(30) Priority: 05.10.2010 JP 2010225651; 05.10.2010 JP 2010225649; 05.10.2010 JP 2010225646
(71) Applicant: Panasonic Corporation, Osaka 571-8501 (JP)
(72) Inventor: KOHNO, Haruhiko, Osaka-shi, Osaka 540-6207 (JP); KAWANO, Yuuzou, Osaka-shi, Osaka 540-6207 (JP); SANADA, Takafumi, Osaka-shi, Osaka 540-6207 (JP)
(74) Representative: Eisenführ, Speiser & Partner
(86) International application number: PCT/JP2011/005460
(87) International publication number: WO 2012/046413

(57) **Abstract**

An endoscope includes an imaging unit capturing an image of an object; an imaging holder holding the imaging unit; two systems of driving force transmission mechanisms each including a pair of driving rods having distal ends connected in a diagonal position to each other relative to the imaging holder; a driving apparatus disposed on a base end side of the driving rods and driving forward and backward at least one of the driving rods in each of the driving force transmission mechanisms; and a cover member extending from a base member of the driving apparatus and covering at least a portion of the imaging unit, the imaging holder, and the driving force transmission mechanisms. The imaging unit rotates around two different axes according to forward and backward movement of the driving rods.

## Description

### FIELD OF THE INVENTION

The present invention relates to an endoscope capturing an inside of an object that cannot be observed directly from the outside, particularly to an endoscope capable of changing a viewing direction during image capturing.

### BACKGROUND ART

In order to change a viewing direction in capturing an image (observation) with a rigid endoscope having a highly rigid insertion portion, it is conventionally necessary to displace the entire insertion portion inside an object or to prepare in advance a plurality of insertion portions having different viewing directions to be exchanged appropriately for use.

To address this circumstance, a technology is known in which an operable curved portion is provided at a distal end of an insertion portion to which a solid-state image sensing device is attached, thus allowing easy change of a viewing direction during image capturing (refer to Patent Document 1).

Another technology is known in which a solid-state image sensing device housed in a distal end of an insertion portion is rotatably held around one predetermined axis and is rotated through a wire or rod inserted from a driving apparatus (operation portion) to the insertion portion, thus allowing change of a viewing direction during image capturing without bending the insertion portion (namely, without requiring a surrounding space) (refer to Patent Documents 2 and 3).

### PRIOR ART DOCUMENTS

### PATENT DOCUMENTS

[Patent Document 1] Japanese Patent Laid-open Publication No. 2005-342010
[Patent Document 2] Japanese Patent Laid-open Publication No. H7-327916
[Patent Document 3] Japanese Patent Laid-open Publication No. 2006-95137

### SUMMARY OF THE INVENTION

### TASKS TO BE ACCOMPLISHED BY THE INVENTION

With the conventional technology disclosed in Patent Documents 2 and 3 above, however, there is a circumstance in which, although the viewing direction can be changed during image capturing, the change of the viewing direction is limited to a rotation range of the solid-state image sensing device around one fixed axis. To address the circumstance, it is considered that the solid-state image sensing device be rotated around two axes (i.e., the number of rotation axes is increased) to allow change of the viewing direction over a wider range. In the configuration of the conventional technology, however, a rotation radius is increased, thus leading to an increase in the size of the apparatus (i.e., increase in the diameter of the insertion portion).

In addition, the apparatus configuration is extremely complex since it requires increasing the number of driving force transmission mechanisms (such as wires and rods) for forward and backward driving and driving apparatuses (such as motors) as well as increasing the number of rotation axes. Furthermore, it is desirable that a drive board for driving a solid-state image sensing device used in an endoscope be provided proximate thereto in view of input of a reference clock for signal processing to the solid-state image sensing device without attenuation and conversion of parallel data output from the solid-state image sensing device to serial data for reduction of a transmission wiring amount. In the case where the solid-state image sensing device is rotated around two axes to allow change of the viewing direction over a wider range during image capturing in the conventional technology disclosed in Patent Documents 2 and 3, there are circumstances in which the size of the apparatus is increased (the diameter of the insertion portion is increased) and the apparatus configuration around the solid-state image sensing device is too complex to dispose a drive board proximate thereto.

In view of the circumstances above, a main advantage of the present invention is to provide an endoscope capable of changing a viewing direction during image capturing over a wide range without an increase in size of an apparatus (specifically, increase in a diameter of an insertion portion). Another advantage is to provide an endoscope capable of reducing the number of driving rods required for forward and backward driving to rotate an imaging unit.
Still another advantage is to provide an endoscope capable of stably driving a solid-state image sensing device and enhancing reliability of image processing.

### MEANS TO ACCOMPLISH THE TASK

An aspect of the present invention provides an endoscope including an imaging unit capturing an image of an object; an imaging holder holding the imaging unit; two systems of driving force transmission mechanisms each including a pair of driving rods having distal ends connected in a diagonal position to each other relative to the imaging holder; a driving apparatus disposed on a base end side of the driving rods and driving forward and backward at least one of the driving rods in each of the driving force transmission mechanisms; and a cover member extending from a base member of the driving apparatus and covering at least a portion of the imaging unit, the imaging holder, and the driving force transmission mechanisms, wherein the imaging unit rotates around two different axes according to forward and backward movement of the driving rods.

Another aspect of the present invention provides an endoscope including an imaging unit capturing an image of an object; an imaging holder holding the imaging unit; two systems of driving force transmission mechanisms each including a pair of driving rods having distal ends connected in a diagonal position to each other relative to the imaging holder; a driving apparatus disposed on a base end portion of the driving rods and driving forward and backward at least one of the driving rods in each of the driving force transmission mechanisms; a support shaft extending from a base member side of the driving apparatus toward the imaging unit; a relay holder mounted on the support shaft and supporting intermediate portions of the driving rods; and a cover member covering at least a portion of the imaging unit, the imaging holder, the driving force transmission mechanisms, the support shaft, and the relay holder, wherein the other driving rod in each of the driving force transmission mechanisms is not driven by the driving apparatus and the base end portion thereof is connected to the base member side through an elastic member, and the imaging unit rotates around two different axes according to forward and backward movement of the one driving rod.

Still another aspect of the present invention provides an endoscope including an imaging unit having an imaging device for capturing an image of an object; an imaging holder holding the imaging unit; a drive board driving the imaging device; two systems of driving force transmission mechanisms each including a pair of driving rods having distal ends connected in a diagonal position to each other relative to the imaging holder; a driving apparatus disposed on a base end side of the driving rods and driving forward and backward at least one of the driving rods in each of the driving force transmission mechanisms; a support shaft extending from a base member side of the driving apparatus toward the imaging unit; a relay holder mounted on the support shaft and supporting intermediate portions of the driving rods; and a cover member covering at least a portion of the imaging unit, the imaging holder, the drive board, the driving force transmission mechanisms, the support shaft, and the relay holder, wherein the imaging unit rotates around two different axes according to forward and backward movement of the driving rods and the drive board is disposed between the imaging unit and the relay holder in the cover member.

According to the present invention, the imaging unit can be rotated around two axes without requiring a large space. Thus, an excellent effect is exhibited in which a viewing direction during image capturing can be changed over a wider range without increasing the size of the apparatus (i.e., increasing the diameter of the cover member forming the insertion portion). In addition, use of the elastic member can reduce the number of driving rods driven forward and backward to rotate the imaging unit. Furthermore, the drive board is disposed proximate to the imaging device, thus allowing stable driving of the imaging device and improving the reliability of the apparatus.

### BRIEF DESCRIPTION OF THE DRAWING

Fig. 1 is a side view of an endoscope according to an embodiment;
Fig. 2 is an exploded perspective view of an external casing of an insertion portion according to the embodiment;
Fig. 3 is a cross-sectional view of the endoscope according to the embodiment;
Fig. 4 is a perspective view of a main portion of an inside of the insertion portion according to the embodiment;
Fig. 5 is an exploded perspective view of a vicinity of an imaging unit according to the embodiment;
Fig. 6 is an exploded perspective view of a driving force transmission mechanism according to the embodiment;
Fig. 7 is a perspective view of a driving apparatus built in a main body according to the embodiment;
Figs. 8A and 8B are each a side view of a main portion illustrating operation of the driving force transmission mechanism according to the embodiment;
Figs. 9A and 9B are each a schematic view illustrating a method of operating the driving force transmission mechanism according to the embodiment;
Figs. 10A and 10B each illustrate a method of folding a flexible cable connecting a solid-state image sensing device and its drive board according to the embodiment;
Fig. 11 is a schematic view illustrating a positional relationship between the drive board of the solid-state image sensing device and driving rods according to the embodiment;
Fig. 12 is a block diagram illustrating a configuration of a control system of the driving apparatus according to the embodiment; and
Fig. 13 is a timing chart illustrating an exemplary operation of the driving apparatus according to the embodiment.

### DETAILED DESCRIPTION OF PREFERRED EMBODIMENTS

A first aspect of the present invention provides an endoscope including an imaging unit capturing an image of an object; an imaging holder holding the imaging unit; two systems of driving force transmission mechanisms each including a pair of driving rods having distal ends connected in a diagonal position to each other relative to the imaging holder; a driving apparatus disposed on a base end side of the driving rods and driving forward and backward at least one of the driving rods in each of the driving force transmission mechanisms; and a cover member extending from a base member of the driving apparatus and covering at least a portion of the imaging unit, the imaging holder, and the driving force transmission mechanisms. The imaging unit rotates around two different axes according to forward and backward movement of the driving rods.

According to the first aspect, the imaging unit can be rotated around the two axes without requiring a large space, thus allowing change of a viewing direction during image capturing over a wider range without increasing the size of the apparatus.

In a second aspect of the present invention, the endoscope according to the first aspect is further configured to include a support shaft extending from the base member side toward the imaging unit; and a relay holder mounted on the support shaft and supporting intermediate portions of the driving rods.

According to the second aspect, the forward and backward movement of the driving rods is guided by the relay holder. Thus, the imaging unit can be stably rotated around the two axes in a small space regardless of the lengths of the driving rods.

In a third aspect of the present invention, the endoscope according to the second aspect is further configured such that the relay holder is tiltably mounted relative to the support shaft.

According to the third aspect, the driving rods can function as a linking mechanism that links the relay holder and the imaging holder, thus enabling the imaging unit to rotate around the two axes in a further stable manner in a small space.

In a fourth aspect of the present invention, the endoscope according to the third aspect is further configured such that the relay holder is mounted on the support shaft through a ball joint.

Thereby, the relay holder can tilt simply and stably.

In a fifth aspect of the present invention, the endoscope according to the first aspect is further configured such that the two axes are mutually orthogonalized.

Thereby, a pan/tilt function can be easily available during image capturing.

In a sixth aspect of the present invention, the endoscope according to the first aspect is further configured such that a distal end portion of the cover member is provided with a projection having a hemispherical surface transmissive to light from the object, and the imaging holder is slidably in contact with an internal surface of the distal end portion of the cover member as the imaging unit rotates.

According to the sixth aspect, rotation of the imaging holder is guided by the internal peripheral surface of the distal end projection of the cover member, and thus the imaging unit can be rotated stably around the two axes in a small space.

In a seventh aspect of the present invention, the endoscope according to the sixth aspect is further configured such that the imaging holder has a plurality of ribs slidably in contact with the internal surface of the distal end portion of the cover member.

According to the seventh aspect, the rotation allows simple and stable sliding of the imaging holder relative to the internal surface of the distal end portion of the cover member.

In an eighth aspect of the present invention, the endoscope according to the second aspect is further configured such that the other driving rod in each of the driving force transmission mechanisms is not driven by the driving apparatus and the base end portion thereof is connected to the base member side through an elastic member.

According to the eighth aspect, the imaging unit can be rotated around the two axes without requiring a large space, thus allowing change of a viewing direction during image capturing over a wider range without increasing the size of the apparatus. Furthermore, use of the elastic member can reduce the number of driving rods driven forward and backward to rotate the imaging unit. In this case, the driving force may be provided to move the driving rods either forward or backward by tensile force or compression force of the elastic member. In addition, the elastic member prevents instability of the imaging unit in rotation and the relay holder eliminates any effect from the force of the elastic member on the imaging unit.

In a ninth aspect of the present invention, the endoscope according to the eighth aspect is further configured such that the other driving rod holds a predetermined tensile force between the intermediate portion connected to the relay holder and the base end portion connected to the base member side, the tensile force being exerted by the elastic member; and the one driving rod holds another predetermined tensile force between the intermediate portion connected to the relay holder and the base end portion connected to the base member side, the tensile force being exerted by the tensile force applied to the relay holder.

According to the ninth aspect, the elastic member prevents instability of the imaging unit in rotation and the relay holder eliminates any effect from the force of the elastic member on the imaging unit.

In a tenth aspect of the present invention, the endoscope according to the eighth aspect is further configured such that the elastic member is a tensile spring.

According to the tenth aspect, a force in a buckling direction is prevented from being exerted on the driving rods, and thus the imaging unit can be stably rotated around the two axes.

In an eleventh aspect of the present invention, the endoscope according to the second aspect is further configured to include a drive board driving the imaging device; the drive board being disposed between the imaging unit and the relay holder inside the cover member.

According to the eleventh aspect, the imaging unit can be rotated around the two axes without requiring a large space, thus allowing change of a viewing direction during image capturing over a wider range without increasing the size of the apparatus. Furthermore, the drive board is disposed proximate to the imaging device, thus allowing stable driving of the imaging device and improving the reliability of the apparatus.

In a twelfth aspect of the present invention, the endoscope according to the eleventh aspect is further configured such that the drive board is surrounded externally by the driving rods, a board surface of the drive board is disposed in a direction not crossing an extending direction of the cover member, and a longitudinal direction of the drive board viewed from the extending direction of the cover member is disposed not crossing the positions of the driving rods.

According to the twelfth aspect, the driving rods are prevented from interfering with an installation space of the drive board even in a case where a distance between the driving rods is reduced in rotation of the imaging unit.

In a thirteenth aspect of the present invention, the endoscope according to the eleventh aspect is further configured such that the drive board is connected to the imaging unit through a flat flexible cable folded so as to deform according to rotation of the imaging unit.

According to the thirteenth aspect, the flexible cable connecting the rotatable imaging unit and the fixed drive board can be readily deformed in any direction with no locally concentrated stress in rotation of the imaging unit.

An embodiment of the present invention is explained below with reference to the drawings.

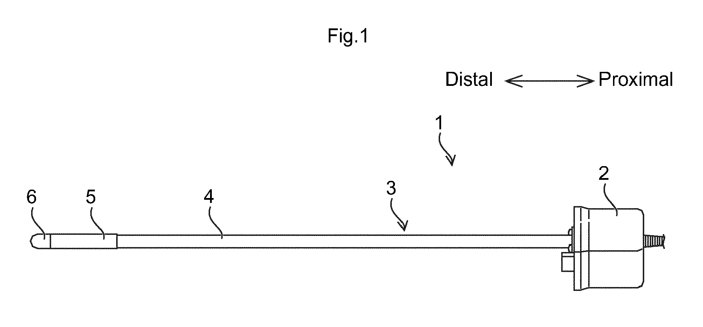

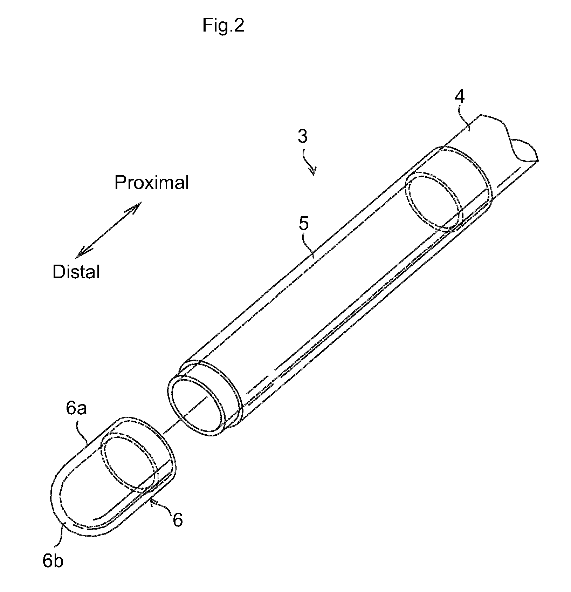

Fig. 1 is a side view of an endoscope 1 according to the embodiment. Fig. 2 is an exploded perspective view of an external casing of an insertion portion 3.

The endoscope 1 is a rigid scope for medical or industrial use. The endoscope 1 primarily has a main body 2 and the insertion portion 3 extending forward from the main body 2. The insertion portion 3 has a small diameter (e.g., an external diameter of 8 mm) and high rigidity so as not to be bent easily. The insertion portion 3 is inserted into an object (not shown in the drawing, e.g., patient's body).

The external casing (cover member) of the insertion portion 3 demarcates a hermetically-sealed internal space. The external casing includes a protection tube 4, an intermediate cover 5, and a distal end cover 6, the protection tube 4 being composed of a metal having a proximal end (base end) fixed to the main body 2, the intermediate cover 5 being composed of a cylindrical resin or glass connected to a distal end (front end) of the protection tube 4, the distal end cover 6 being formed of a glass connected to a distal end of the intermediate cover 5. The distal end cover 6 functions as a light-transmissive imaging window. As shown in Fig. 2, the distal end cover 6 has a cylindrical portion 6a and a distal end projection 6b, the cylindrical portion 6a being fitted and attached to the distal end portion of the intermediate cover 5, the distal end projection 6b having a hemispherical surface and extending from the distal end of the cylindrical portion 6a.

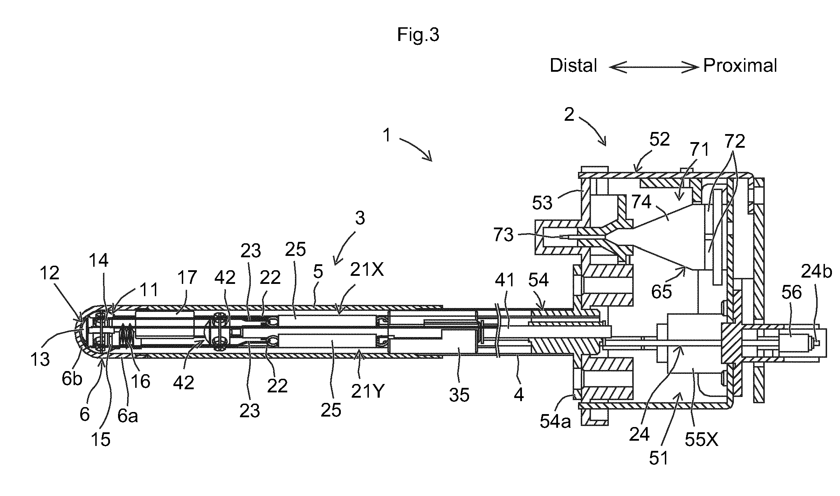

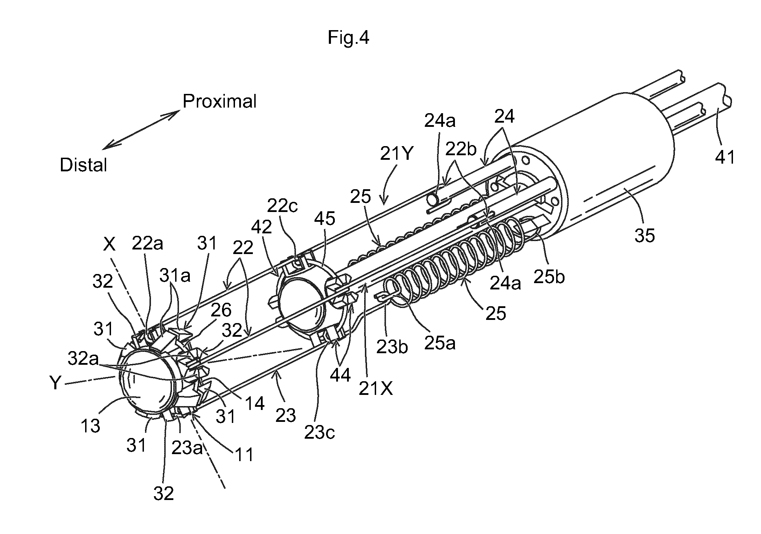

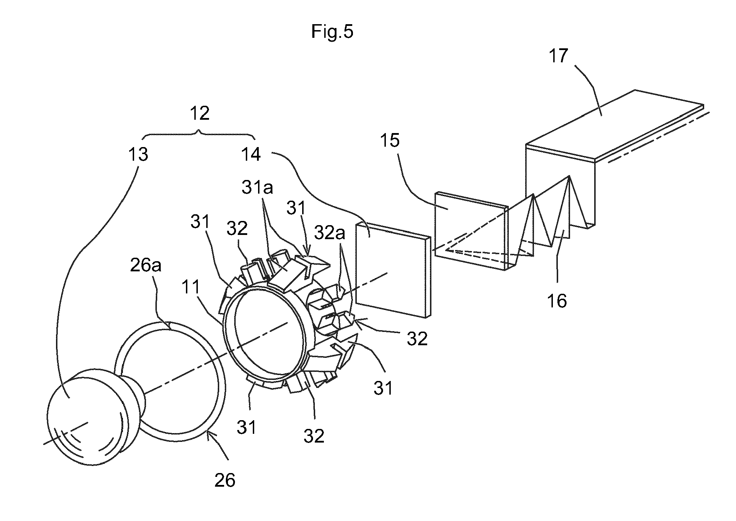

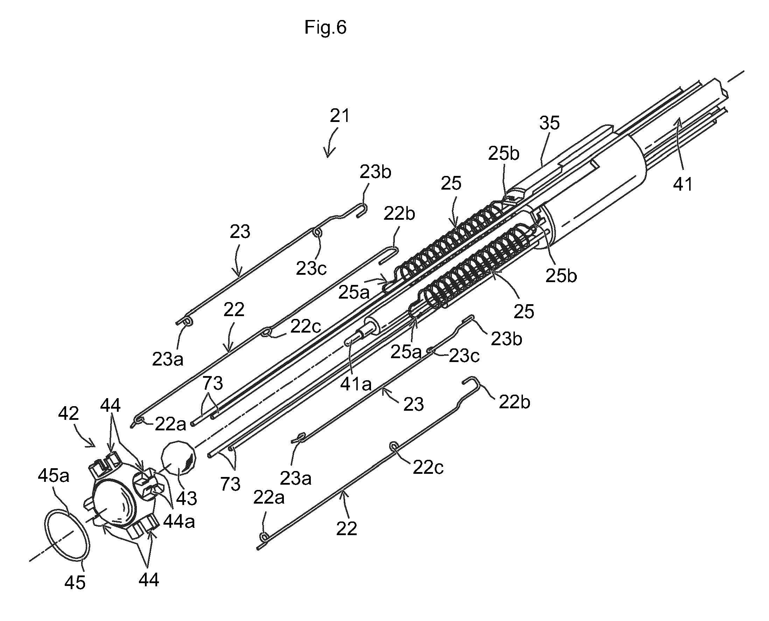

Fig. 3 is a cross-sectional view of the endoscope 1. Fig. 4 is a perspective view of a main portion of an inside of the insertion portion 3. Fig. 5 is an exploded perspective view of a vicinity of an imaging unit 12. Fig. 6 is an exploded perspective view of a driving force transmission mechanism 21.

As shown in Figs. 3 and 4, the imaging unit 12 is disposed in the distal end of the internal space of the insertion portion 3, the imaging unit 12 being rotatably held around two axes (X axis and Y axis in Fig. 4) by an imaging holder 11 and capturing an image of an object while changing a viewing direction. The imaging unit 12 has an objective lens system 13 and a solid-state image sensing device 14, the objective lens system 13 being composed of one or more optical lenses, the solid-state image sensing device 14 being disposed in the rear of the objective lens system 13 and forming an image of light from the lenses on a light receiving surface. The imaging unit 12 has a viewing angle of approximately 170°. In the description below, components associated with rotation around the two axes (X axis and Y axis) of the imaging unit 12 are each referred to with a suffix (X or Y) following a component name or a reference numeral for differentiation. In the solid-state image sensing device 14, its main scanning direction and sub-scanning direction correspond to the X axis and Y axis, respectively

As shown in Fig. 5, the imaging holder 11 has a cylindrical main body to which the rear portion of the objective lens system 13 is fitted and inserted. The solid-state image sensing device 14 is attached to the rear side of the main body. The solid-state image sensing device 14 may be a known image sensor composed of, for example, a CMOS (Complementary Metal Oxide Semiconductor).

A rear surface of the solid-state image sensing device 14 is bonded to and directly supported by a joint 15 of a flexible cable 16 through a BGA (Ball Grid Array) connection. The joint 15 is connected to a drive board 17 (refer to Fig. 3) by the flat flexible cable 16 that transmits and receives a variety of signals and supplies power. A voltage conversion circuit for power to drive the solid-state image sensing device 14 and a clock generation circuit are provided on the drive board 17. An AD converter may be mounted on the drive board 17 in a case where the solid-state image sensing device 14 has no AD converter therein. The drive board 17 is positioned between the imaging unit 12 in the anteroposterior direction and a relay holder 42 hereinafter described, and is fixed to the internal peripheral surface of the intermediate cover 5. A cable (not shown in the drawing) that transmits and receives captured image data and the like is provided between the drive board 17 and an image processor (not shown in the drawing) on the main body 2 side.

As shown in Figure 4, two systems of driving force transmission mechanisms 21X and 21 Y that rotate the imaging unit 12 in the two axial directions are provided in the internal space of the insertion portion 3. The two systems of driving force transmission mechanisms 21X and 21Y have structures similar to each other. A pair of a first driving rod 22 and a second driving rod 23 are provided in each structure, the first driving rod 22 and the second driving rod 23 having distal ends connected in a diagonal position to each other relative to the imaging holder 11 and extending in the anteroposterior direction.

The first and second driving rods 22 and 23 are bar-shaped members each composed of a so-called flexible spring steel. As shown in Fig. 6, the first and second driving rods 22 and 23 have distal end portions 22a and 23a, respectively, each of which is wound once and formed into a substantially annular shape (spiral shape). As shown in Fig. 4, a ring member 26 is inserted through openings of the distal end portions 22a and 23a, the ring member 26 being also composed of a spring steel and being attached around the external periphery of the main body of the imaging holder 11. The ring member 26 is loosely held inside the openings of the distal end portions 22a and 23a.

As shown in Fig. 5, four ribs 31 are provided on the external periphery of the main body of the imaging holder 11 at equal distances in the circumferential direction. Furthermore, four support guides 32 are provided between the ribs 31 to position the distal end portions 22a and 23a of the first and second driving rods 22 and 23, respectively. A groove extending in the circumferential direction is provided in an intermediate portion in the anteroposterior direction of each of the ribs 31. The ring member 26 is fitted into the groove. As shown in Fig. 5, the ring member 26 is composed of a circular metal ring having one cut portion 26a. The ring member 26 is temporarily deformed to disengage the two ends of the cut portion 26a when inserted into the openings of the distal end portions 22a and 23a of the first and second driving rods 22 and 23.

Each of the support guides 32 is composed of a pair of guide pieces 32a opposed to each other in the circumferential direction. In an intermediate portion in the anteroposterior direction of each of the guide pieces 32a, a groove is provided to which the ring member 26 is fitted, similar to the ribs 31. The distal end portions 22a and 23a of the first and second driving rods 22 and 23, respectively, are provided in a space between the guide pieces 32a forming a pair. Thus, the circumferential movement of the distal end portions 22a and 23a is limited within a predetermined range.

The two ends of the cut portion 26a of the ring member 26 are disengaged in advance and the openings of the distal end portions 22a and 23a (two each as shown in Fig. 6) are inserted through the ring member 26, are positioned with an appropriate jig, and are fitted to the imaging holder 11 by being moved in the anteroposterior direction along an optical axis (dashed-dotted line in Fig. 5). Thereby, the ring member 26 is fitted into the grooves of the ribs 31 and the support guides 32. Concurrently, the distal end portions 22a and 23a are fitted in a loose state between the guide pieces 32a of the support guides 32 to the ring member 26. Thus, the imaging holder 11 is engaged so as to be displaced according to the forward and backward movement of the first and second driving rods 22 and 23.

As another method of engaging the imaging holder 11 with the distal end portions 22a and 23a of the first and second driving rods 22 and 23, respectively, the ring member 26 may be fitted into the grooves of the ribs 31 and the support guides 32 in advance; the ring member 26 may be rotated in the circumferential direction of the imaging holder 11 in a state where the cut portions 26a are slightly separated; and the distal end portions 22a and 23a may be each inserted to the position of the guide pieces 32a at the point where the separated portion reaches the position of the guide pieces 32a. In this case, it is preferred that the ring member 26 be configured such that the cut portions 26a are separated in a non-load state and that the separation distance be at least equal to or greater than the diameter of the first and second driving rods 22 and 23.

In each of the driving force transmission mechanisms 21, a hook-shaped proximal end portion 22b of the first driving rod 22 is connected with a distal end portion 24a of a metal motor connection rod 24 extending in the anteroposterior direction, as shown in Fig. 4. The motor connection rod 24 is movably inserted through a guide hole of a cylindrical guide member 35 fixed and supported by a support shaft 41 hereinafter described. A proximal end portion 24b thereof reaches as far as the main body 2, as shown in Fig. 3. A distal end portion 25a of a tension spring (elastic member) 25 extending in the anteroposterior direction is connected to a hook-shaped proximal end portion 23b of the second driving rod 23. A proximal end portion 25b of the tension spring 25 is fixed to a front surface of the guide member 35.

In addition, each of the driving force transmission mechanisms 21 has a base end fixed to the main body 2, as shown in Fig. 3. The support shaft 41 extends from the main body 2 in the anteroposterior direction along the center axis of the insertion portion 3. A hemispherical relay holder 42 is attached to a distal end of the support shaft 41 to support the first and second driving rods 22 and 23. The guide member 35 is fixed to an intermediate portion of the support shaft 41.

More specifically, as shown in Fig. 6, a spherical ball member 43 is attached to a distal end portion 41a of the support shaft 41. The ball member 43 is slidably accommodated in a receiver (not shown in the drawing) having a spherical sliding surface provided in a rear portion or inside of the relay holder 42, thereby providing a ball joint. The relay holder 42 is tiltably held at the distal end of the support shaft 41 through the ball joint. The maximum external diameter of the relay holder 42 is set smaller than the internal diameter of the external casing (intermediate cover 5 herein) of the insertion portion 3, so as to prevent the relay holder 42 from coming into contact with the external casing of the insertion portion 3 while being tilted.

The first and second driving rods 22 and 23 have intermediate portions 22c and 23c, respectively, each of which is wound once and formed into a substantially annular shape, similar to the distal end portions 22a and 23a, respectively. A ring member 45 is inserted through openings of the intermediate portions 22c and 23c, the ring member 45 being attached to the external periphery of the main body of the relay holder 42. Similarly to the ring member 26, the ring member 45 is composed of a circular spring steel having one cut portion 45a and is loosely held in the openings of the intermediate portions 22c and 23 c.

Four guides 44 are provided on the external periphery of the main body of the relay holder 42, the four guides 44 having similar structures to the support guides 32 of the imaging holder 11. Each of the guides 44 is composed of a pair of guide pieces 44a positioning the intermediate portions 22c and 23c of the first and second driving rods 22 and 23, respectively. Each of the guide pieces 44a is provided with a groove to which the ring member 45 is fitted. The relay holder 42 may be provided with ribs, similar to the ribs 31 of the imaging holder 11.

Similarly to the case of the imaging holder 11, two ends of the cut portion 45a of the ring member 45 are disengaged in advance and the openings of the intermediate portions 22c and 23c (two each as shown in Fig. 6) are passed through the ring member 45, are positioned with an appropriate jig, and are fitted to the relay holder 42 by being moved in the anteroposterior direction along an optical axis (dashed-dotted line in Fig. 6). Thereby, the ring member 45 is fitted into the grooves of the guides 44. Concurrently, the intermediate portions 22c and 23c are fitted in a loose state between the guide pieces 44a of each of the guides 44 to the ring member 45. Thus, the relay holder 42 is tiltably engaged according to the forward and backward movement of the first and second driving rods 22 and 23.

To engage the relay holder 41 with the intermediate portions 22c and 23c of the first and second driving rods 22 and 23, respectively, and, the "another method" of engaging the imaging holder 11 with the distal end portions 22a and 23a of the first and second driving rods 22 and 23, respectively, may be applied as it is.

Thus, the configuration connecting the intermediate portions 22c and 23c of the first and second driving rods 22 and 23, respectively, with the relay holder 42 allows the driving rods 22 and 23 to function as a linking mechanism connecting the relay holder 42 and the imaging holder 11, thus achieving stable rotation of the imaging unit 12 around the two axes (X and Y axes in Fig. 4) in a small space.

In rotation of the imaging unit 12 by the driving force transmission mechanism 21X, the rotation axis (X axis in Fig. 4) substantially coincides with an axis passing through the distal end portions 22a and 23a of the first and second driving rods 22 and 23, respectively, paired in the driving force transmission mechanism 21Y (the other system). Similarly, in rotation of the imaging unit 12 by the driving force transmission mechanism 21Y, the rotation axis (Y axis in Fig. 4) substantially coincides with an axis passing through the distal end portions 22a and 23a of the first and second driving rods 22 and 23, respectively, paired in the driving force transmission mechanism 21X. Thus, the X axis and Y axis are not fixed to the positional relationship shown in Fig. 4, but are displaced in rotation of the imaging unit 12 (i.e., according to the displacement or deformation of the driving rods 22 and 23). The X axis and the Y axis may be orthogonalized to each other so that a pan/tilt function is readily available in image capturing. The two axes, however, do not have to be orthogonalized. They may be simply crossed, not orthogonalized, or may be twisted with each other depending on the situation.

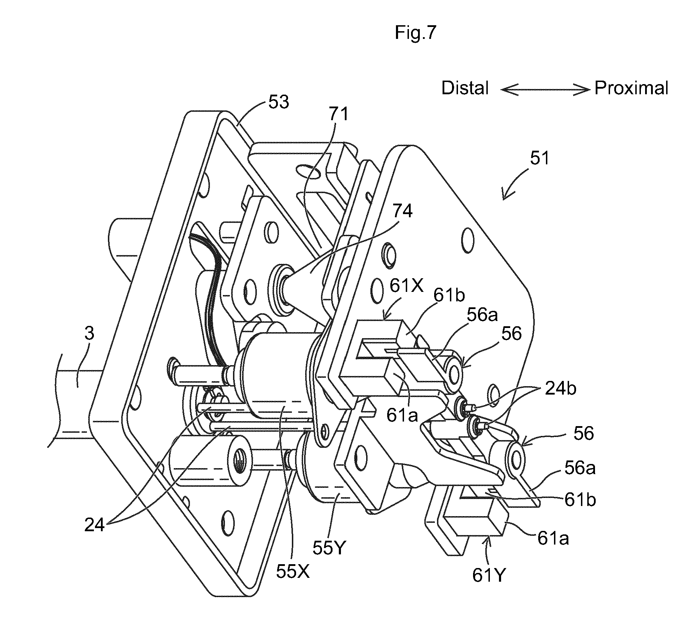

Fig. 7 is a perspective view of a driving apparatus 51 built into the main body 2. As shown, supplemented with Fig. 3, the main body 2 of the endoscope 1 includes the driving apparatus 51 that drives forward and backward the first and second driving rods 22 and 23 in the driving force transmission mechanisms 21. A base member 53 forming a front surface of a case 52 of the driving apparatus 51 is provided with a flange 54a of a fixing member 54 to which the proximal end of the insertion portion 3 (protection tube 4) is attached. Two electric motors 55X and 55Y are provided in the case 52 to provide driving force to the driving force transmission mechanisms 21X and 21 Y, respectively.

The electric motors 55X and 55Y are direct acting stepping motors each having a motor shaft (screw shaft; not shown in the drawing) to convert rotation movement into linear movement. The stepping motors are driven by what is generally-called a micro-step drive. The proximal end portions 24b of the motor connection rods 24 in the respective driving force transmission mechanisms 21 are provided adjacent to the motor shafts of the electric motors 55X and 55Y extending in the anteroposterior direction and are connected to the motor shafts through connecting members 56. With the configuration, the first driving rod 22 and the second driving rod 23 in the respective driving force transmission mechanisms 21 can move forward and backward along the axis (anteroposterior direction) of the insertion portion 3 in a substantially linear way according to the rotation amount of the electric motors 55X and 55Y (i.e., movement amount of the motor shafts).

The driving apparatus 51 is further provided with two origin sensors 61X and 61Y that detect original positions of the motor shafts (i.e., motor connection rods 24) of the electric motors 55X and 55Y, respectively. The origin sensors 61X and 61Y are PI (photo interrupter) sensors each having a light emitter 61 a and a light receiver 61 b opposed to each other. A shutter piece 56a blocking the light from the light emitter 61a projects from each of the connecting members 56. With this configuration, the shutter piece 56a is inserted between the light emitter 61a and the light receiver 61b as the motor shaft to which the connecting member 56 is attached moves, so as to recognize a position at which the light from the light emitter 61a is completely blocked as the original position of the motor shaft.

The main body 2 shown in Fig. 3 includes an illumination apparatus 71 for image capturing. The illumination apparatus 71 has a plurality of LEDs (light emitting diodes) 72 and a transparent resin light guide 74 provided in front of the LEDs 72 and guiding the light output from the LEDs 72 to four optical fiber cables 73. According to a purpose of image capturing, the LEDs 72 can selectively output white light, ultraviolet light, or infrared light; or can output the light simultaneously. As shown in Fig. 6, the optical fiber cables 73 extend proximate to the imaging unit 12 through the inside of the insertion portion 3, and are thus capable of radiating light to an object from a distal end of the cable.

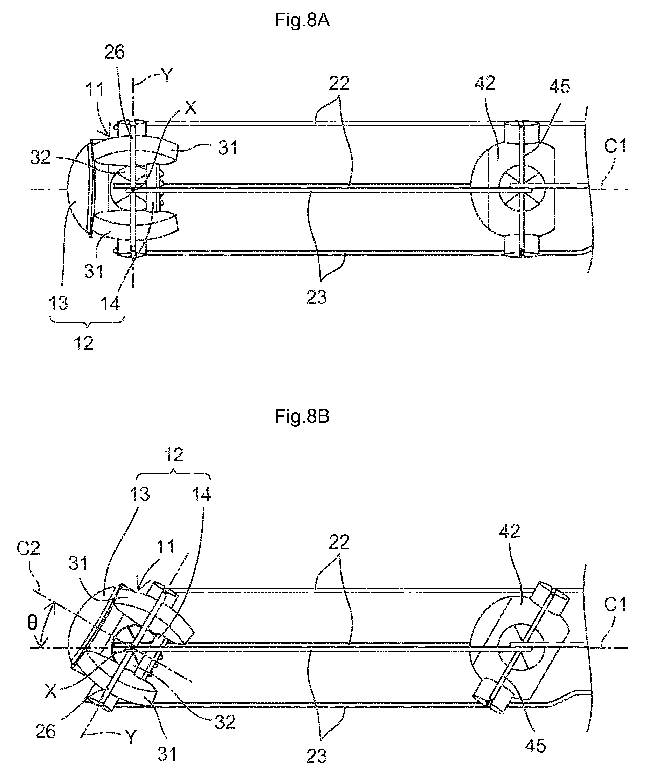

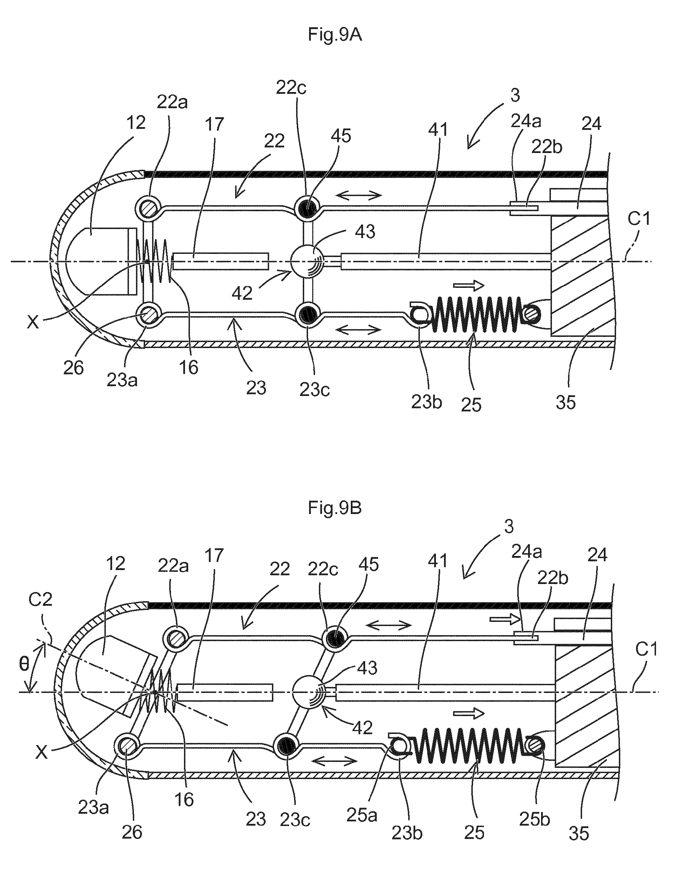

Figs. 8A and 8B are each a side view of a main portion illustrating operation of the driving force transmission mechanism 21. Figs. 9A and 9B are each a schematic view illustrating a method of operating the driving force transmission mechanism 21.

As shown in Fig. 8A, the viewing direction of the imaging unit 12 is directed forward along the center axis C1 of the insertion portion in the endoscope 1 in the initial state. As shown in Fig. 9A, a backward force by the tension spring 25 (urging force of the spring) is exerted on the proximal end portion 23b of the second driving rod 23, thus causing a predetermined tensile force between the intermediate portion 23c and the proximal end portion 23 b of the second driving rod 23 supported by the ring member 45 of the relay holder 42. The tensile force exerted by the tensile spring 25 is transmitted to the intermediate portion 22c through the relay holder 42, thus also causing a tensile force between the intermediate portion 22c and the proximal end portion 22b of the first driving rod 22 supported by the ring member 45 of the relay holder 42.

To change the viewing direction of the endoscope 1, the electric motor 55X shown in Fig. 3 is activated to retract the motor connection rod 24. As shown in Fig. 8B, the imaging unit 12 is then rotated around the X axis to change the viewing direction. As shown in Fig. 4, the external peripheral surfaces 31a of the ribs 31 of the imaging holder 11 each have a curvature identical to that of the internal peripheral surface of the distal end projection 6b (refer to Fig. 2) of the distal end cover 6. In rotation of the imaging unit 12, the external peripheral surfaces 31a of the ribs 31 are slidably in contact with the internal peripheral surface of the distal end projection 6b to guide the rotation. An example is shown herein in which a tilt angle θ of the center axis C2 of the image capturing unit 12 is 30° relative to the center axis C1 of the insertion unit. The tilt angle θ may be set to any value within a predetermined range (e.g., 0° ≤ θ ≤ 35 °).

At this time, a backward force (driving force of the electric motor 55X) is exerted on the proximal end portion 22b of the first driving rod 22 through the motor connection rod 24, as shown in Fig. 9B. Thereby, the relay holder 42 is tilted to retract the distal end portion 22a of the first driving rod 22 and advance the distal end portion 23a of the second driving rod 23. Accordingly, the driving force is exerted on the ring member 26 of the imaging holder 11 and the imaging unit 12 is rotated around the X axis against the urging force of the tensile spring 25. In the above operation to change the viewing direction, predetermined tensile forces are generated between the intermediate portion 23c and the proximal end portion 23 b of the second driving rod 23 and between the intermediate portion 22c and the proximal end portion 22b of the first driving rod 22.

This configuration allows the tensile forces to be exerted constantly on the first and second driving rods 22 and 23, thus preventing a force from being exerted thereon in a buckling direction and enhancing the reliability of the driving force transmission mechanism 21. Furthermore, the relay holder 42 can thus be tilted smoothly.

The operation example of moving the motor connection rod 24 backward is illustrated herein. The motor connection rod 24 may also be moved forward. In this case, similar tensile forces as above are also exerted on the first and second driving rods 22 and 23 by the tensile spring 25. Furthermore, only the operation around the X axis is illustrated, but the operation may be performed around the Y axis in a similar manner. In addition, the electric motors 55X and 55Y may be operated concurrently or sequentially to rotate around the two axes. For instance, the driving speed of each of the electric motors 55X and 55Y is changed in a sinusoidal shape and phases thereof are controlled. Thereby, the center of the image capturing range can be moved on an arc or an operation can be performed to draw what is generally-called a Lissajous figure.

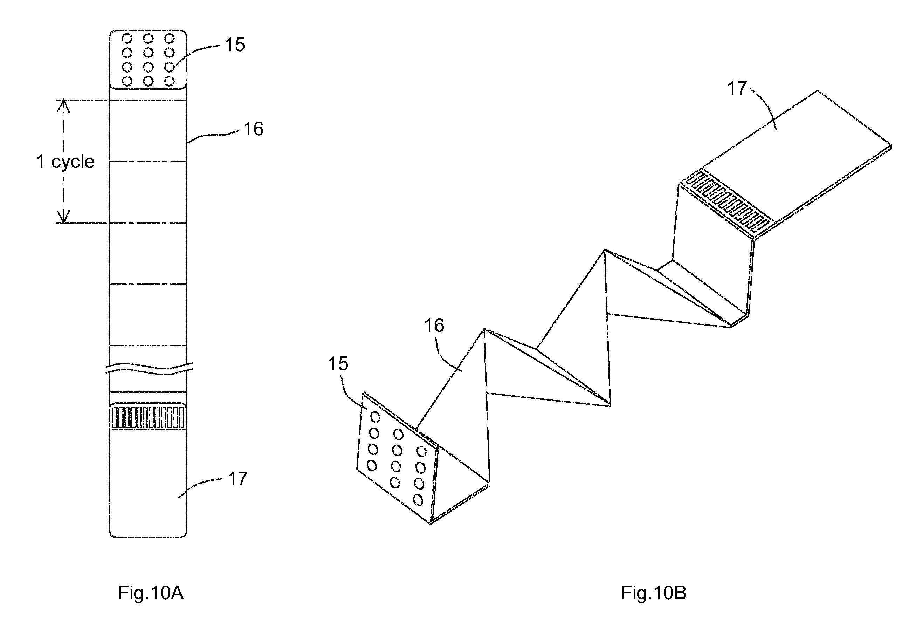

Fig. 10A illustrates a method of folding the flexible cable 16 connecting the solid-state image sensing device 14 and its drive board 17. Fig. 10B illustrates a folded state of the flexible cable 16.

As described above, the imaging unit 12 is rotatable while the drive board 17 is fixed to the intermediate cover 5 of the insertion portion 3. It is thus preferred that the flexible cable 16 connecting both components readily deform in any direction with no locally concentrated stress during rotation of the imaging unit 12. It is also preferred that the length be as short as possible in view of noise proofing and attenuation of clock signals output from the drive board 17 to the solid-state image sensing device 14.

The flexible cable 16 is folded into a shape shown in Fig. 10B. Specifically, as shown in Fig. 10A, portions disposed at predetermined intervals in a longitudinal direction (indicated by dashed-dotted lines in Fig. 10A) are mountain-folded, while portions each connecting one side end of a mountain-folded portion and the other side end of an adjacent mountain-folded portion (indicated by medium broken lines in Fig. 10A) are valley-folded. Thereby, the flexible cable 16 can be easily bent in a parallel direction in addition to an orthogonal direction to the drive board 17. The flexible cable 16 bendable in any direction is thus provided with a very short length.

Bending the flexible cable 16 within one cycle shown in Fig. 10A allows deformation at least in the two-axis directions. Providing two or more cycles allows the flexible cable 16 to extend and contract according to a distance change (initial position error) of the operation portion (imaging unit 12) and the fixed portion (drive board 17). In view of improvement in reliability, it is also preferred to provide two or more cycles.

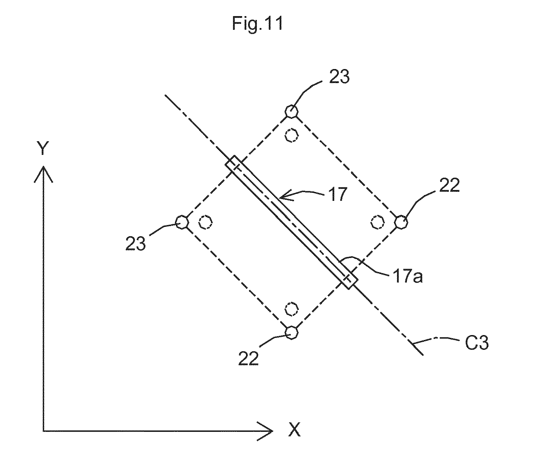

Fig. 11 is a schematic view illustrating a positional relationship between the drive board 17 of the solid-state image sensing device 14 and the driving rods 22 and 23. In rotation of the imaging unit 12 as shown in Figs. 8A to 9B, positions of the first and second driving rods 22 and 23 which externally surround the drive board 17 may be displaced inward as viewed from the axis direction of the insertion portion 3, in accordance with an increase in the rotation angle, as shown with dashed-two dotted circles in Fig. 11.

As shown in Fig. 11, it is thus preferable that a board surface 17a of the drive board 17 be provided so as not to cross the axis direction of the insertion portion 3 (perpendicular direction to the paper surface of Fig. 11) and that an axis line C3 of the drive board 17 in the longitudinal direction viewed from the extending direction of the insertion portion be provided so as not to overlap the positions of the driving rods 22 and 23. In Fig. 11, the drive board 17 is disposed such that angles defined by the axis line C3 and the X axis and by the axis line C3 and the Y axis are both 45°. Thereby, the imaging unit 12 in rotation can be prevented from interfering with the installation space of the drive board 17 even in a case where the distance between the driving rods 22 and 23 is reduced.

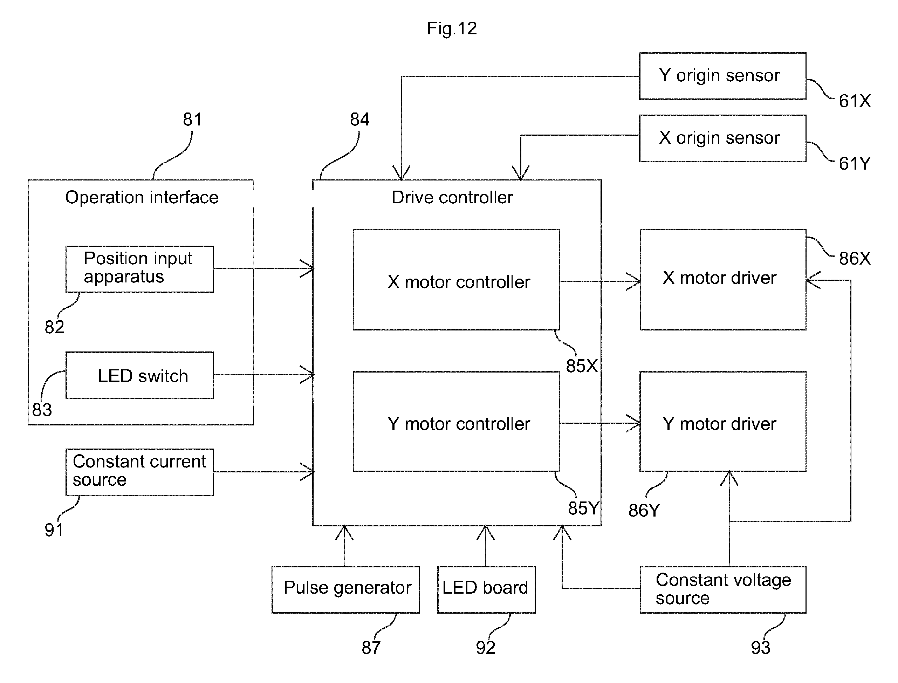

Fig. 12 is a block diagram illustrating a configuration of a control system of the driving apparatus 51. An operation interface 81 provided in the driving apparatus 51 has a position input apparatus 82 and an LED switch 83, the position input apparatus 82 being provided for an operator to adjust the viewing direction, the LED switch 83 turning on and off an LED and selecting a type of light (white light, ultraviolet light, and infrared light) to be radiated. The position input apparatus 82 may be composed of a joystick or a trackball.

A drive controller 84 has motor controllers 85X and 85Y which control the electric motors 55X and 55Y, respectively (refer to Fig. 7). Based on position control signals including position information input from the position input apparatus 82, the motor controllers 85X and 85Y output to motor drivers 86X and 86Y, respectively, drive control signals to control a rotation direction and a rotation amount of each of the electric motors 55X and 55Y, respectively. The motor drivers 86X and 86Y drive the electric motors 55X and 55Y, respectively, based on the input drive control signals.

Based on original position signals input from the origin sensors 61X and 61Y, the drive controller 84 recognizes that the electric motors 55X and 55Y, respectively, are at their original positions. A clock input from a pulse generator 87 is supplied to a CPU (Central Processing Unit; not shown in the drawing) at the drive controller 84, so as to coordinate the entire control timing. Furthermore, the clock is used as a reference clock of driving pulses for the electric motors 55X and 55Y or to set timings of sample-and-hold and flip-flop for analog output from the origin sensors 61X and 61Y. Power supplied from a constant current source 91 to the drive controller 84 is input to an LED board 92 to turn the LEDs 72 on. In addition, control voltage is input from a constant voltage source 93 to the drive controller 84 and the motor drivers 86X and 86Y.

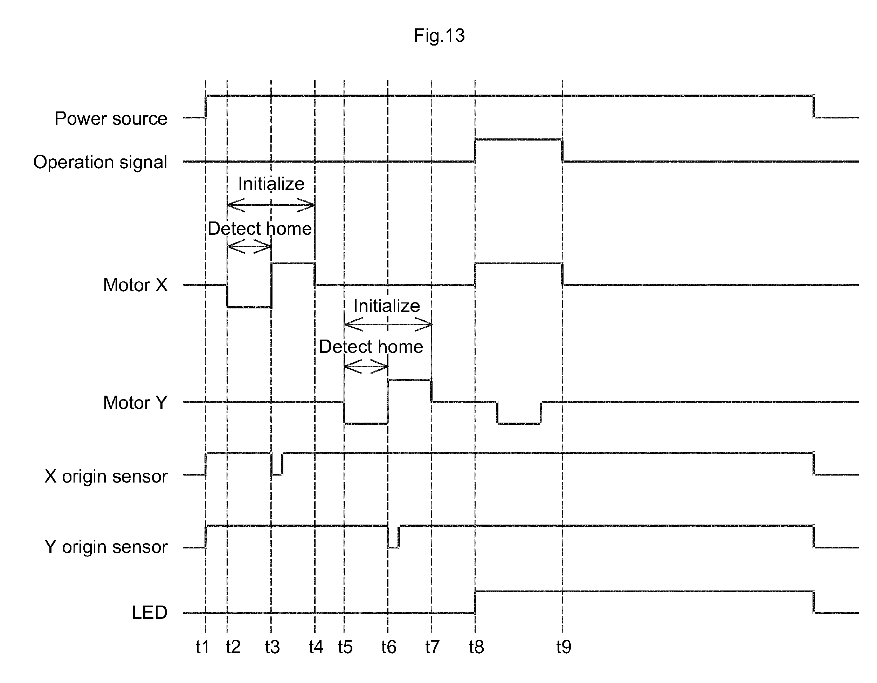

Fig. 13 is a timing chart illustrating an exemplary operation of the driving apparatus 51. The CPU (not shown in the drawing) controls each timing illustrated in Fig. 13.

The power of the endoscope is first turned on at a time t1, and then the X origin sensor 61X is monitored by the CPU. Thereafter, initialization of the motor X (electric motor 55X) is started at a time t2. In the initialization, the X origin sensor 61X moves a motor shaft in a predetermined direction (forward herein) until it detects an original position, and thereby a home detection is executed (time t3). Subsequently, the motor shaft of the motor X is moved in the reverse direction back to the original position, and thereby the initialization is completed (time t4). The motor Y (electric motor 55Y) is then initialized from times t5 to t7 in a similar manner to the motor X.

When the initialization of the motors X and Y is completed, the imaging unit 12 starts to capture an image (obtain captured image data) and an operator is allowed to operate the viewing direction of the endoscope using the operation interface. Operation signals are input by the operator at times t8 and t9, the operation signals being associated with an instruction to change the viewing direction. Then, the motors X and Y execute a predetermined operation based on the operation signals. The captured image data (image signals output from the solid-state image sensing device 14) obtained in the image capturing are transmitted to a signal processor (not shown in the drawing) connected to the main body. After predetermined image processing, such as color correction and gamma correction, the data are transmitted to a monitor and the captured image is displayed.

As described above, the endoscope 1 provided with the two systems of driving force transmission mechanisms 21X and 21Y, each of which includes a pair of the driving rods 22 and 23, can rotate the imaging unit 12 around the two axes without requiring a large space. Thus, the endoscope 1 allows change of the viewing direction over a wider range in image capturing without increasing the size of the apparatus (i.e., increasing the size of the external casing of the insertion portion 3).

In the endoscope 1, the intermediate portions 22c and 23c of the driving rods 22 and 23, respectively, are supported by the relay holder 42 which is tiltably attached to the support shaft 41. Thus, the imaging unit 12 can be stably rotated around the two axes in a small space regardless of the lengths of the driving rods 22 and 23.

The endoscope 1 has a configuration in which the drive board 17 is disposed in the space between the imaging unit 12 and the relay holder. Thereby, the drive board 17 can be disposed proximate to the solid-state image sensing device 14 so as to stably drive the solid-state image sensing device 14 for improved reliability in image processing.

In the endoscope 1, the second driving rod 23 is not driven by the electric motors 55X and 55Y. Instead, the rear end portion 23b of the second driving rod 23 is connected to the base member 53 (guide member 35) through the tensile spring 25, thus reducing the number of driving rods (corresponding to the first driving rod 22) driven forward and backward to rotate the imaging unit 12. Furthermore, the tensile spring 25 prevents instability of the imaging unit 12 during rotation. An additional advantage is that the relay holder 42 can eliminate any effect from the urging force of the tensile spring 25 on the imaging unit 12.

The present invention is explained based on specific embodiments. The embodiments, however, are merely exemplary and the present invention is not limited to these. For example, in a case where the endoscope is used in an environment free from intrusion of liquid into the insertion portion, the external casing of the insertion portion is not necessarily required to demarcate the hermetically-sealed internal space. Instead, a configuration may suffice that covers at least a portion of the imaging unit or the driving power transmission mechanism.

The relay holder may have at least a function to support the driving rod. For example, the intermediate portion of the driving rod may be provided in a straight line shape, and the relay holder fixed to the support shaft may have a guide hole through which the straight intermediate portion is inserted.

The first driving rod and the motor connection rod in the driving force transmission mechanism may be integrally provided (for instance, the base end of the first driving rod in the embodiment is extended to eliminate the motor connection rod). Alternatively, the first driving rod may be composed of a plurality of rods. For instance, two rods may be disposed in the front and rear of the relay holder, and the two rods may be indirectly connected through the relay holder (ring member) (i.e., a base end of the front driving rod and a front end of the rear driving rod terminate at a portion supported by the relay holder.)

In medical use of the endoscope, at least the insertion portion may be provided to be separable using a known adapter for sterilization of the insertion portion.

The driving force to rotate the imaging unit does not necessarily have to be generated by the electric motor. A known configuration may be employed in which an operator manually generates the driving force.

The elastic member used in the driving force transmission mechanism is not limited to the tensile spring, but another known member may be employed. In some cases, a compression spring may be used to generate a force in the reverse direction from the tensile spring.

The elastic member (tensile spring) is not necessarily used in the driving force transmission mechanism. All rods may be driven by an electric motor or the like. In this case, paired driving rods are preferably driven in directions opposite to each other for the same displacement amount.

Not all components of the endoscope of the present invention according to the embodiment are necessarily required. The components may be appropriately eliminated or selected without deviating from the scope of the present invention.

### INDUSTRIAL APPLICABILITY

The endoscope according to the present invention is capable of changing a viewing direction during image capturing over a wide range without an increase in size of an apparatus (specifically, increase in a diameter of an insertion portion), and thus, is useful as an endoscope capable of changing a viewing direction during image capturing.

## Claims

1. An endoscope comprising:
an imaging unit capturing an image of an object;
an imaging holder holding the imaging unit;
two systems of driving force transmission mechanisms each including a pair of driving rods having distal ends connected in a diagonal position to each other relative to the imaging holder;
a driving apparatus disposed on a base end side of the driving rods and driving forward and backward at least one of the driving rods in each of the driving force transmission mechanisms; and
a cover member extending from a base member of the driving apparatus and covering at least a portion of the imaging unit, the imaging holder, and the driving force transmission mechanisms, wherein
the imaging unit rotates around two different axes according to forward and backward movement of the driving rods.

2. The endoscope according to claim 1, further comprising:
a support shaft extending from the base member side toward the imaging unit; and
a relay holder mounted on the support shaft and supporting intermediate portions of the driving rods.

3. The endoscope according to claim 2, wherein the relay holder is tiltably mounted relative to the support shaft.

4. The endoscope according to claim 3, wherein the relay holder is mounted on the support shaft through a ball joint.

5. The endoscope according to claim 1, wherein the two axes are mutually orthogonalized.

6. The endoscope according to claim 1, wherein
a distal end portion of the cover member is provided with a projection having a hemispherical surface transmissive to light from the object, and
the imaging holder is slidably in contact with an internal surface of the distal end portion of the cover member as the imaging unit rotates.

7. The endoscope according to claim 6, wherein the imaging holder has a plurality of ribs slidably in contact with the internal surface of the distal end portion of the cover member.

8. The endoscope according to claim 2, wherein the other driving rod in each of the driving force transmission mechanisms is not driven by the driving apparatus and the base end portion thereof is connected to the base member side through an elastic member.

9. The endoscope according to claim 8, wherein the other driving rod holds a predetermined tensile force between the intermediate portion connected to the relay holder and the base end portion connected to the base member side, the tensile force being exerted by the elastic member; and the one driving rod holds another predetermined tensile force between the intermediate portion connected to the relay holder and the base end portion connected to the base member side, the tensile force being exerted by the tensile force applied to the relay holder.

10. The endoscope according to claim 8, wherein the elastic member is a tensile spring.

11. The endoscope according to claim 2 further comprising a drive board driving the imaging device, the drive board being disposed between the imaging unit and the relay holder inside the cover member.

12. The endoscope according to claim 11, wherein the drive board is surrounded externally by the driving rods, a board surface of the drive board is disposed in a direction not crossing an extending direction of the cover member, and a longitudinal direction of the drive board viewed from the extending direction of the cover member is disposed not crossing the positions of the driving rods.

13. The endoscope according to claim 11, wherein the drive board is connected to the imaging unit through a flat flexible cable folded so as to deform according to rotation of the imaging unit.
